# EUROPEAN PATENT APPLICATION

(11) **EP 2 106 794 A1**
(43) Date of publication of application: **07.10.2009**
(21) Application number: 08154054.4
(22) Date of filing: 04.04.2008
(51) Int. Cl.: A61K 31/355, A61K 31/714, A61K 38/16, A61K 45/06, A61P 17/00

(54) **Ubiquitin alone or in combination for use in the treatment of sweet itch.**

(71) Applicant: Eden Horse B.V., Willemstad, Curacao (AN)
(72) Inventor: van der Windt, Arie-Dirk, 9665 BN Oude Pekela (NL)
(74) Representative: Hatzmann, Martin

(57) **Abstract**

The present invention relates to the field of preventive and therapeutic veterinary medicine. In particular, it relates to compositions and methods for the prophylaxis or treatment of sweet itch in equine species, horses, ponies, donkeys. Provided is the use of ubiquitin, or ubiquitin-related molecule, for the manufacture of a medicament for the prevention or treatment of sweet itch in an equine mammal. Also provided is a composition comprising pharmaceutically active quantities of ubiquitin or ubiquitin-related molecule and tocopherol, together with an acceptable carrier.

## Description

The present invention relates to the field of preventive and therapeutic veterinary medicine. In particular, it relates to compositions and methods for the prophylaxis or treatment of sweet itch in equine species, horses, ponies, donkeys.

Sweet itch is a distressing skin condition that affects thousands of horses across the world, where it is also known as Queensland Itch, Summer Itch, Summer Eczema and Seasonal Dermatitis. It occurs in varying degrees of severity in horses of various breeds, and there appears to be a genetic link. It is particularly common in certain breeds, including Icelandic, Arabian and Friesian horses. Most horses develop symptoms at 3 or 4 years of age. Coat colour does not seem to be an issue.

Sweet itch is a seasonal condition (spring through fall) caused by a hypersensitivity to the bites of the Culicoides flies (also called midges and 'no-see-ums'). After being bitten, some horses are allergic to a substance in the saliva of the fly, which causes a local reaction. This in turn causes intense irritation with severe itching, which the horse tries to relieve by rubbing and biting itself. The culicoides midges feed primarily at dusk and dawn, and tend to feed on the horse at specific sites, particularly around the head, tail head, withers, and base of the mane; however other areas, including the chest, back and rump can also be affected. The midges like to breed on wet land, and around rivers, lakes and standing water, so susceptible animals kept close to these conditions are more likely to be affected.

Affected animals are very itchy and distressed, and rub and bite themselves intensely. The fly bites form blisters, which can weep, causing crusting, scabs and scaling. Prolonged rubbing and biting results in, among others, hair loss and damage to the skin, with sometimes bleeding open sores. Occasionally, secondary bacterial infections can occur. It is also not uncommon for horses to rub off their mane and upper tail hair. In the long term, skin thickening and loss of hair pigmentation may occur.

Thus far, it has been tried mainly to prevent the symptoms of sweet itch by protecting the animal from the flies. Suggested measures include:
* Removing standing water.
* Stabling the animal from 4pm until 8am during summer months.
* Using regular fly repellents.
* Using rugs to provide some protection.
* Fitting a very fine fly screen on stable door to protect the animal while it is stabled.

However, despite taking such precautions to protect an animal from the culicoides flies, it can often be extremely difficult to prevent the symptoms of Sweet Itch, and without treatment, the condition tends to worsen each year. There is presently no known cure for Sweet Itch.

In view of the apparent absence of a treatment of sweet itch-related problems in equine animals, it is an object of this invention to provide such a treatment. Particularly, an object for this invention is to provide a treatment, which effectively prevents and/or treats at least some of the symptoms of sweet itch, preventing further distress to the animal. A further object is the provision of a therapeutic or prophylactic composition, e.g. a food supplement that can be easily administered to equine mammals, such horses, ponies and donkeys, which is free of side effects, and which has a beneficial result against sweet itch.

The present inventors surprisingly discovered that the systemic (e.g. intravenous or oral) administration of ubiquitin is beneficial to alleviate most, if not all, symptoms of sweet itch. It calms down the animal's distress, soothes the irritated skin and relieves the itching. It also promotes rapid healing of any sores or open wounds, and encourages quick hair regrowth. Improvements were observed within 3 days following start of the treatment, even in the most severe cases. Very good and long-lasting effects were obtained when ubiquitin was used in combination with vitamin B12, also known as cobalamine.

The invention accordingly relates to the use of ubiquitin or ubiquitin-related molecule (functional fragment, analog or derivative thereof), for the manufacture of a medicament for the prevention or treatment of sweet itch in an equine mammal. Also provided is a composition comprising pharmaceutically active quantities of ubiquitin or ubiquitin-related molecule and vitamin B12, together with a pharmaceutically acceptable carrier. Furthermore, the invention provides a method for treating or preventing sweet itch in an equine mammal, comprising administering to said animal an effective dosage amount of a composition according to the invention.

Ubiquitin is a small protein that occurs in all eukaryotic cells. Its main known function is to mark other proteins for destruction, known as proteolysis. At least four ubiquitin molecules attach to a lysine residue on the condemned protein, in a process called polyubiquitination, and the protein then moves to a proteasome, a barrel-shaped structure where the proteolysis occurs. Apparently, at least four ubiquitins are required on a substrate protein in order for the proteasome to bind and therefore degrade the substrate, though there are examples of non-ubiquitinated proteins being targeted to the proteasome. Ubiquitin can also mark transmembrane proteins (for example, receptors) for removal from membranes and fulfill several signalling roles within the cell. Monoubiquitination has been associated with targeting of membrane proteins to the lysosome, for example. Ubiquitin consists of 76 amino acids and has a molecular mass of about 8.5 kDa. Key features include its C-terminal tail and the Lys residues. It is highly conserved among eukaryotic species: Human and yeast ubiquitin share 96 % sequence identity.

Because ubiquitin can be covalently linked to either ubiquitin itself or other proteins and its structure is highly conserved, a similar action is expected for other ubiquitin-related molecules. As used herein, the term "ubiquitin-related molecule" refers to functional fragments, derivatives and homologs of ubiquitin. Progress in the study of the ubiquitin system has revealed the existence of multiple molecules that are structurally related to ubiquitin (reviewed in Johnson, P. R., and Hochstrasser, M. (1997) Trends Cell Biol. 7, 408-413 and Tanaka, K., Suzuki, T., and Chiba, T. (1998) Mol. Cells 8, 503-512). They include, for example: UBLs ; ubiquitin-like proteins (e.g. SUMO1); proteins with homology to ubiquitin in amino acid composition or structure; proteins containing ubiquitin-like domains. As used herein, the term "fragment" is intended to mean a contiguous segment of at least 5, preferably at least 10 amino acid residues of ubiquitin. Of particular interest are peptide fragments corresponding to the ubiquitin (50-59) sequence (LEDGRTLSDY) and the pentapeptide DGRTL (Ubiquitin 52-56). As used herein, the term"derivative"is intended to mean a natural or synthetic modification of ubiquitin. As used herein, the term"analog" is intended to mean proteins with homology to ubiquitin in amino acid sequence or three dimensional structure (UbLs; ubiquitin- like domains).

Ubiquitin can be administered to an equine animal by any means known to be suitable to those of skill in the art, including oral, intraperitoneal, intranasal, intravenous, subcutaneous, intradermal, and intramuscular administration.

Effective amounts of ubiquitin and related compounds (i. e. analogs and derivatives) are amounts that are sufficient to bring about an efficacious clinical effect, and can be determined by those of skill in the art by routine experimentation. In general, an effective daily dosage of ubiquitin for the purposes of this invention is expected to be about 1 microgram to 100 milligram/ kg body weight, preferably 10 microgram to 50 milligram/ kg body weight, and more preferably 20 microgram to 25 milligram/ kg body weight. Analogs and derivatives should have similar ranges of efficacy based on their relative molecular weights.

It was surprisingly found that the supplementation of a ubiquitin-containing composition with alpha-tocopherol, also known as vitamin E, has several advantageous effects. It allows for a reduction in the amount of ubiquitin required to exert a therapeutic effect. For example, therapeutic effects on sweet itch of a daily dosage of 50 milligram ubiquitin per kg body weight were similar to those of 50 microgram ubiquitin when combined with tocopherol. Furthermore, the combination of ubiquitin and tocopherol was also found to be even more effective in curing the sweet itch symptoms as compared to ubiquitin alone. Accordingly, in one embodiment the invention provides a composition comprising pharmaceutically active quantities of ubiquitin or ubiquitin-related molecule and alpha-tocopherol, together with an acceptable carrier. For example, a daily dose of about 5 to 2000, preferably 10-1000, more preferably 20-100 µg / kg body weight of ubiquitin is administered together with about 100-1000 IU, preferably 250-600 IU of tocopherol A per animal. In another embodiment, ubiquitin or ubiquitin-related molecule is used in combination with vitamin B12 for the manufacture of a medicament. It was surprisingly found that the addition of vitamin B12 leads to prolonged beneficial effects on the animals treated (see Example 1 herein below). Accordingly, the invention also provides the use of vitamin B12 for the therapeutic or prophylactic treatment of sweet itch. Preferably, vitamin B12 is used in combination with ubiquitin or ubiquitin-related molecule optionally in further combination with alpha tocopherol.

The term "vitamin B12" refers to a group of cobalamin compounds, of which the most frequently used is cyanocobalamin. In the early to mid 1800s, an unrecognized vitamin B12 deficiency was referred to as pernicious anemia because it was almost always fatal. It was not until 1948, though, that researchers finally isolated the active principle in liver now called vitamin B12. Like most of the vitamins, B12 is required as a cofactor for various enzymes. Every DNA-synthesizing cell requires vitamin B12. It facilitates the cyclic metabolism of folic acid, which is essential for thymidine (one of the four DNA bases) synthesis. It also transfers a methyl group from methylfolate, helping to convert homocysteine to methionine. Vitamin B12 is an especially important vitamin for maintaining healthy nerve cells. Vitamin B12 also works closely together with vitamin B9 (folate) to regulate the formation of red blood cells and to help iron function better in the body.

Administration of vitamin B12 to horses is known in the art. For example, a 6 ml oral dose syringe comprising 5000 micrograms of Vitamin B12 is available as nutritional food supplement from Horse Supplies Direct Pty Ltd under the trade name Micro B12 5x Oral gel. It is advertised as being beneficial for a diverse array of normal functions in animals, including growth, health, feed efficiency and reproduction; formation of red blood cells and carbohydrate and fat metabolism. However, the benefits of administering vitamin B12 for sweet itch prevention or treatment in an equine animal is not disclosed or suggested in the art, let alone in combination with ubiquitin.

In one embodiment, vitamin B12 is used in an amount between 10 and 1000 IU per animal, preferably 100 to 800 IU, more preferably from about 300-600 IU, like 400 or 500 IU, per equine animal. A further aspect relates to a composition comprising pharmaceutically active quantities of ubiquitin or ubiquitin-related molecule and vitamin B12, together with a pharmaceutically acceptable carrier. Preferably, the composition further comprises at least one additional vitamin, preferably a vitamin from the vitamin B complex. The B vitamins are eight water-soluble vitamins that play important roles in cell metabolism. Historically, the B vitamins were once thought to be a single vitamin, referred to as Vitamin B. Later research showed that they are chemically distinct vitamins that often coexist in the same foods. Supplements containing all eight B vitamins are generally referred to as a vitamin B complex. The inclusion of vitamin B6 (also known as pyridoxine), and/or vitamin B11 (folic acid) is particularly preferred. In one embodiment, the invention provides a composition comprising, in addition to ubiquitin and vitamin B12, vitamin B6, vitamin B12, vitamin B1 (thiamine) and vitamin B2 (riboflavin). The relative amounts of the various components can vary. In one embodiment, the relative ratio of tocopherol to vitamin B12 ranges from 1: 2 to to 2: 1, preferably about 1: 1, as expressed in international units (IU).
Provided is a composition, comprising per daily dosage unit, about 30-50 microgram ubiquitin or ubiquitin-related molecule, 200-500 IU tocopherol, and 200-500 IU vitamin B12, optionally together with vitamin B6 and/or vitamin B11.

Other useful ingredients include minerals and salts, such as selenium and/or zinc.

A composition of the invention is advantageously used as nutritional supplement for equine mammals, in particular horses, more in particular horses suffering from sweet itch or being at risk of developing sweet itch symptoms.

In one embodiment, there is provided an equine food supplement comprising an effective amount of ubiquitin or ubiquitin-related molecule and vitamin B12, optionally together with an acceptable carrier, such as a binder. In another embodiment, the invention provides a composition for use as a medication. Said pharmaceutical composition comprises pharmaceutically active quantities of ubiquitin or ubiquitin-related molecule and vitamin B12, together with a pharmaceutically acceptable carrier. It was surprisingly observed that horses receiving a daily dose of the anti-sweet itch composition comprising tocopherol, ubiquitin and vitamin B12 also displayed significant improvements with respect to symptoms associated with arthritis, such as fluid retention in the joints and overall stiffness. Accordingly, a composition according to the invention also finds its application in the treatment or prevention of arthritis, in particular osteoarthritis, in an equine mammal.

Also provided is a package or container comprising a composition according to the invention. Exemplary packages include sachets, plastic containers and syringes. The package may contain instructions for use.

A composition may thus be formulated for administration via the oral, intraperitoneal, intranasal, intravenous, subcutaneous, intradermal, and intramuscular route. In view of the ease of administration, a composition is preferably formulated for non-invasive, e.g. oral administration. Accordingly, in one embodiment the invention provides the use of ubiquitin or ubiquitin-related molecule, for the manufacture of a medicament formulated for oral administration.

For oral administration as a nutritional dietary supplement, therapeutic or prophylactic agent, the composition may be provided as a dispersible powder or granule, tablet, pellet, hard or soft capsule, emulsion, aqueous or oil suspension, syrup or elixir. Compositions intended for oral use may be prepared in accordance with any method known in the art for the manufacturing of nutritional supplement compositions and such compositions may contain one or more of the following components: preservatives, sweeteners, flavoring agents and coloring agents. The flavoring agents and sweetening will enhance the palatability of the preparation. Known flavoring agents for horses are artificial apple and oatmeal flavouring agents.

Tablets containing ubiquitin, preferably in combination with vitamin B12, in admixture with non-toxic pharmaceutically acceptable excipients suitable for tablet manufacture are acceptable. Such excipients include inert diluents such as calcium carbonate, sodium carbonate, lactose, calcium phosphate or sodium phosphate, granulating and disintegrating agents, such as corn starch or alginic acid, binding agents such as starch, gelatin or acacia; and lubricating agents such as magnesium stearate, stearic acid or talc. Tablets may be uncoated or may be coated by known techniques to delay disintegration and absorption in the gastrointestinal tract and thereby provide a sustained action over a longer period of time. For example, a time delay material such as glyceryl monostearate or glyceryl distearate alone or with a wax may be employed. The use of enteric coating is also contemplated.

Formulations for oral use may also be presented as hard gelatin capsules wherein the active ingredient is mixed with an inert solid diluent, for example calcium carbonate, calcium phosphate or kaolin, or as soft gelatin capsules wherein the active ingredient is mixed with water or an oil medium, such as peanut oil, liquid paraffin or olive oil.

Aqueous suspensions may contain the powder material of the invention in admixture with excipients suitable for the manufacture of aqueous suspensions. Such excipients include suspending agents, dispersing or wetting agents, one or more preservatives, one or more coloring agents, one or more flavoring agents and one or more sweetening agents such as sucrose or saccharin.

Oil suspensions may be formulated by suspending the active ingredient(s) in a vegetable oil, such as arachis oil, olive oil, sesame oil or coconut oil, or in a mineral oil such as liquid paraffin. The oil suspension may contain a thickening agent, such as beeswax, hard paraffin or cetyl alcohol. Sweeting agents, such as those set forth above, and flavoring agents may be added to provide a palatable oral preparation. These compositions may be preserved by an added antioxidant such as ascorbic acid.

Dispersible powders and granules of the invention suitable for preparation of an aqueous suspension by the addition of water provide the active ingredient in admixture with a dispersing or wetting agent, a suspending agent, and one or more preservatives. Additional excipients, for example, sweetening, flavoring and coloring agents may also be present. Syrups and elixirs may be formulated with sweetening agents, such as glycerol, sorbitol or sucrose. Such formulations may also contain a demulcent, a preservative, a flavoring agent and/or a coloring agent. The composition in powder form may be administered in accord with the present inventive method and may be mixed with other ingestible forms and consumed in solid, semisolid solution, suspension or emulsion form. It may also be mixed in conjunction or alternatively with pharmaceutically carriers, flavor enhancers, water, suspending agents and emulsifying agents. In a preferred embodiment, the powder is mixed with a citrus juice such as orange, grapefruit or tangerine due to the promotion of connective tissue formation by ascorbic acid. In a preferred essence, the inventive material may also be formulated in a mixture with ascorbic acid.

In a specific embodiment, a composition is formulated in a palatable pellet or granule which is readily accepted by equine animals. The pellets are preferably designed to be eaten out of the feed bucket to make administration to the animal is even easier.

For use as a nutritional dietary supplement, prophylactic or therapeutic agent the composition of this invention can be orally administered in a daily dosage. For horses, for use as a nutritional dietary supplement, prophylactic or therapeutic agent, the composition is preferably orally administered in a daily dosage generally based on the weight of the animal. For each kilogram of animal weight, a preferred dose is from about 10 microgram to about 10 milligram ubiquitin (-related molecule). Alternatively, and more preferably, the daily dose is about 10 microgram to about 100 microgram per kg of animal weight. And still alternatively a most preferred daily dose is from about 25 microgram to about 60 microgram.

The invention is exemplified by the following Examples.

### EXAMPLE 1

This example illustrates the efficacy of a composition comprising ubiquitin in the treatment of sweet itch. Twenty horses of different ages and breed and all diagnosed with sweet itch were included in the study and received ubiquitin in an amount of 20-50 µg / kg bodyweight daily. Most of the horses showed open skin wounds at the onset of the study.
The horses were divided randomly in four different test groups. Group A was treated daily with a single oral dose of supplement comprising ubiquitin. Group B received ubiquitin daily by an intravenous injection. Group C was treated daily with a single oral dose of supplement comprising ubiquitin in combination with vitamin B12 (700 - 1200 IUg per dose). Group D received ubiquitin in combination with vitamin B12 (700 - 1200 IU per dose) daily by an intravenous injection.

Each of the horses was scored at day 3, day 7 and day 14 following onset of the treatment by two individuals for overall behaviour, wound appearance and hair conditions. Scores ranged from 1 to 5, wherein 1 represents a poor condition and 5 a good condition. Criteria for behaviour included itchiness, distress, rubbing and biting. Criteria for wound appearance included extent of open sores, crusting, scabs and scaling. Hair condition was assessed judging the loss of manes and tail hair as well as the brittleness thereof. The average scores are shown in Table 1. A0 indicates the results for group A horses at day 0, A3 indicates the results for group A horses at day 3, and so on. No harmful side effects of the treatment were observed.

**Table 1: Test Results**

| **Test group** | **behaviour** | **wounds** | **hair condition** |
|---|---|---|---|
| **A0** | **1.2** | **1.8** | **2.0** |
| A3 | 2.2 | 1.9 | 2.3 |
| A7 | 3.5 | 2.5 | 3.0 |
| A14 | 3.1 | 3.1 | 3.2 |
| **B0** | **1.3** | **1.6** | **2.2** |
| B3 | 2.3 | 1.9 | 2.4 |
| B7 | 3.7 | 3.7 | 3.2 |
| B14 | 4.2 | 4.1 | 3.5 |
| **C0** | **1.1** | **1.4** | **1.8** |
| C3 | 1.9 | 1.5 | 1.8 |
| C7 | 3.4 | 2.2 | 2.6 |
| C14 | 3.3 | 2.9 | 2.8 |
| **D0** | **1.2** | **1.5** | **1.9** |
| D3 | 2.3 | 1.7 | 2.2 |
| D7 | 3.9 | 3.8 | 2.8 |
| D14 | 4.4 | 4.2 | 3.1 |

In all test groups, in improvement of the animal's condition could be observed after 3 days of treatment. As expected, the more serious the condition at the onset of the study, the longer it took for the wounds to heal. After 7 days, none of the wounds were bleeding anymore. The animals showed clearly less distress and could be used for training again. The effect of vitamin B12 (groups B and D) is illustrated by the higher end score at 14 days. It was furthermore observed that the effects lasted up to at least 4 weeks, whereas in groups A and C some of the animals showed a relapse and started itching again.

### EXAMPLE 2

In a separate experiment, the effect of treatment with ubiquitin in combination with tocopherol, optionally also in combination with vitamin B12, was determined. Twenty four horses of different breed, age, sex and disease severity were included in the study. The horses were treated daily with the agents shown in Table 2. The onset of symptom disappearance following start of the treatment was monitored. For certain horses, improvements could be observed within one week. It was also determined how long the horses remained symptom-free after the treatment had ended. As is clear from Table 2, the inclusion of vitamin B12 significantly prolongs the beneficial effects of the ubiquitin-treatment. The addition of tocopherol allows for a reduction in the therapeutically effective amount of ubiquitin.

### EXAMPLE 3

A palatable oral equine paste is prepared comprising the following ingredients:

Sucrose, propylene glycol, silicon dioxide, water, flavoring Agents (e.g. synthetic oatmeal or apple flavor), sorbic acid (preservative) and ubiquitin (minimum dosage: 1-10 microgram per 15 ml gel). A 15-ml portion of the paste is placed in a plastic disposable syringe.

### Dosage & Administration:

Give daily one tube (15ml) orally on back of tongue. Take care in regards to trachea (wind pipe).

**TABLE 2**

| **Test animal** | **Breed** | **Age** | **Sex** | **Vit. B12 (IU/animal)** | **Ubiquitin (ug/ kg body weight)** | **A- Tocoferol (IU/animal)** | **Treatment period (weeks)** | **Onset disappearance of symptoms** | **Symptom-free period following treatment** |
|---|---|---|---|---|---|---|---|---|---|
| | | | | | | | | | |
| 1 | Frisian | 11 | Gelding | 400 | 50000 | 0 | 8 | 2 weeks | 2 weeks |
| 2 | Frisian | 5 | Mare | 400 | 50 | 400 | 8 | 2 weeks | 4 weeks |
| 3 | Frisian | 6 | Mare | 0 | 50 | 0 | 8 | 5 weeks | no |
| 4 | Frisian | 6 | Mare | 500 | 50 | 500 | 8 | 2 weeks | 2 weeks |
| 5 | Groninger | 12 | Stallion | 500 | 50 | 500 | 8 | 3 days | 5 weeks |
| 6 | Unknown | 23 | Gelding | 500 | 50 | 500 | 8 | 2 weeks | 4 weeks |
| 7 | Fjord | 15 | Mare | 400 | 50 | 450 | 8 | 1 week | 2 months |
| 8 | Frisian | 3 | Stallion | 0 | 50 | 400 | 8 | 1 week | 3 days |
| 9 | Frisian | 5 | Gelding | 400 | 50 | 400 | 10 | 3 weeks | 5 weeks |
| 10 | Frisian | 8 | Mare | 500 | 50 | 400 | 6 | 2 weeks | 8 weeks |
| 11 | Friesian | 12 | Stallion | 0 | 50 | 400 | 8 | 2 weeks | 2 days |
| 12 | Frisian | 12 | Stallion | 500 | 50 | 400 | 5 | 1 week | 4 weeks |
| 13 | Frisian | 2 | Mare | 400 | 50 | 400 | 6 | 1 week | 8 weeks |
| 14 | Frisian | 8 | Mare | 400 | 50 | 400 | 6 | 2 weeks | 8 weeks |
| 15 | Frisian | 10 | Gelding | 0 | 50 | 400 | 10 | 3 weeks | 3 days |
| 16 | Frisian | 12 | Gelding | 500 | 50 | 400 | 10 | 2 months | 2 months |
| 17 | Frisian | 18 | Mare | 0 | 50000 | 0 | 6 | 6 weeks | no |
| 18 | Frisian | 18 | Mare | 400 | 50 | 400 | 6 | 1 week | 3 months |
| 20 | Unknown | 12 | Gelding | 450 | 50 | 400 | 4 | 2 weeks | 2 months |
| 21 | Fjord | 8 | Mare | 0 | 50 | 400 | 8 | 3 weeks | 3 days |
| 22 | Shetland | 23 | Mare | 400 | 50 | 400 | 8 | 4 weeks | 1 month |
| 23 | Shetland | 2 | Stallion | 500 | 50 | 400 | 8 | 3 weeks | 1 month |
| 24 | Frisian | 21 | Mare | 400 | 50 | 400 | 12 | 5 weeks | 1 month |
| 25 | Frisian | 5 | Mare | 400 | 50 | 400 | 8 | 3 weeks | 1 month |
| 26 | Frisian | 4 | Stallion | 500 | 50 | 400 | 6 | 3 weeks | 1 month |
| 27 | Frisian | 6 | Stallion | 0 | 50 | 400 | 6 | 3 weeks | 5 days |

| **Condition before treatment** | | | | | | | | | |
|---|---|---|---|---|---|---|---|---|---|
| **1** Mild eczema | | | | | **15** severe eczema with small superficial wounds (2-5 cm) in the neck | | | | |
| **2** severe eczema | | | | | **16** severe eczema with deep wounds (2-3 cm) on the tail | | | | |
| **3** very severe eczema; symptoms strongly reduced | | | | | **17** very severe eczema, large deep wounds on neck and tail very severe eczema, large deep wounds on neck and tail; same animal as | | | | |
| **4** Same animal as no.3 but different treatment | | | | | **18** no.17 | | | | |
| **5** Mild eczema | | | | | **20** Mild eczema | | | | |
| **6** very severe eczema with 2 cm deep wounds | | | | | **21** Mild eczema | | | | |
| **7** | | | | | **22** very severe eczema, large superficial wounds (3-12 cm) on neck and tail | | | | |
| **8** | | | | | **23** mild eczema with small wounds (1-3 cm) on neck. Son of animal no. 22 | | | | |
| **9** Very severe eczema; large wounds 5-12 cm | | | | | **24** very severe eczema on neck and tail (3-15 cm) | | | | |
| **10** Mild eczema | | | | | **25** Mild eczema, daughter of animal no. 24 | | | | |
| **11** Very severe eczema; no mane | | | | | **26** mild eczema; full brother of animal no. 27 | | | | |
| **12** same animal as no. 11 very severe eczema; no mane | | | | | **27** mild eczema; full brother of animal no. 26 | | | | |
| **13** Mild eczema | | | | | | | | | |
| **14** Intermediary eczema | | | | | | | | | |

## Claims

1. Use of ubiquitin, or ubiquitin-related molecule, for the manufacture of a medicament for the prevention or treatment of sweet itch in an equine mammal.

2. Use according to claim 1, wherein ubiquitin or ubiquitin-related molecule is used in combination with alpha-tocopherol.

3. Use according to claim 1 or 2, wherein said medicament is formulated for administration via the oral, intraperitoneal, intranasal, intravenous, subcutaneous, intradermal, or intramuscular route, preferably via the oral route.

4. Use according to any one of claims 1-3, wherein the ubiquitin or ubiquitin-related is used in an amount between 1 microgram to 100 milligram/ kg body weight, preferably 10 microgram to 50 milligram/ kg body weight, and more preferably 20 microgram to 25 milligram/ kg body weight.

5. Use according to any one of claims 2-4, wherein alpha-tocopherol is used in an amount between 100 and 1000 IU per daily dosage.

6. Use according to any one of the above claims, wherein said medicament is formulated for oral administration, preferably in the form of a palatable powder, paste, granule or pellet.

7. A composition comprising pharmaceutically active quantities of ubiquitin or ubiquitin-related molecule and alpha-tocopherol, together with an acceptable carrier.

8. Composition according to claim 7 , further comprising at least one additional vitamin, preferably a vitamin from the vitamin B complex, more preferably vitamin B12.

9. Composition according to claim 8, wherein the relative ratio of tocopherol to vitamin B12 ranges from 1: 2 to to 2: 1, preferably about 1: 1, as expressed in international units (IU).

10. Composition as claimed in any of claims 7-9, comprising per daily dosage unit about 30-50 microgramubiquitin or ubiquitin-related molecule, 200-500 IU tocopherol, and 200-500 IU vitamin B12, optionally together with vitamin B6 and/or vitamin B11.

11. Composition as claimed in any of claims 7-10 for use as a nutritional supplement for equine mammals, preferably horses.

12. Composition as claimed in any of claims 7-10 for use as a medication.

13. Package comprising a composition according to any of claims 7-12.

14. A method for treating or preventing sweet itch in an equine mammal, comprising administering to said animal an effective dosage amount of a composition according to any one of claims 7-10.

15. Method according to claim 14, wherein for a horse, for each kg of body weight, said effective dosage amount is between about 1 and 10,000 microgram of ubiquitin or ubiquitin-related molecule daily.

16. The method of claim 14 or 15, wherein the composition is in the form of a powder, paste, gel, granule or pellet.
